# EUROPEAN PATENT APPLICATION

(11) **EP 4 397 744 A1**
(43) Date of publication of application: **10.07.2024**
(21) Application number: 22863870.6
(22) Date of filing: 09.03.2022
(51) Int. Cl.: C12M 1/02, C12M 1/32, C12M 1/36

(54) **SAMPLING DEVICE AND PROGRAM**

(30) Priority: 31.08.2021 JP 2021141479
(71) Applicant: Shimadzu Corporation, Nakagyo-ku, Kyoto-shi, Kyoto 604-8511 (JP)
(72) Inventor: HASUNUMA, Tomohisa, Kobe-shi, Hyogo 657-8501 (JP)
(74) Representative: Müller-Boré & Partner Patentanwälte PartG mbB
(86) International application number: PCT/JP2022/010161
(87) International publication number: WO 2023/032280

(57) **Abstract**

A controller of a sampling apparatus controls a stirrer to operate at a basic speed during a period of circulation of a liquid in a container through a circulation flow path (step S10). The stirrer thus stirs the liquid in the container. The controller controls a flow path switching unit to switch a flow path after lapse of a given time period since lowering in operation speed of the stirrer from the basic speed (step S18). The liquid circulated in the circulation flow path thus flows out to a branch flow path.

## Description

### TECHNICAL FIELD

The present disclosure relates to a sampling apparatus and a program for sampling a liquid in a container.

### BACKGROUND ART

As described in PTL 1, an apparatus that cultures cells of microorganisms or the like by adjusting a dissolved oxygen concentration while a culture medium in a container is stirred has been known.

### CITATION LIST

### PATENT LITERATURE

PTL 1: WO2020/017407

### SUMMARY OF INVENTION

### TECHNICAL PROBLEM

In the apparatus disclosed in PTL 1, a ratio of gas in a liquid (a culture medium or the like) sampled by suction may be varied, and a solution may not accurately be suctioned and split.

The present disclosure was made to solve such a problem, and an object thereof is to provide a technique for accurately sampling a liquid by maintaining an amount of dissolved gas in the sampled liquid constant.

### SOLUTION TO PROBLEM

A first aspect of the present disclosure relates to a sampling apparatus that samples a liquid in a container. The sampling apparatus includes a circulation mechanism that circulates the liquid in the container via a circulation flow path by leading out the liquid from the container into the circulation flow path and introducing the liquid from the circulation flow path into the container, a stirrer provided in the container, the stirrer stirring the liquid in the container, a flow path switching unit that is provided in the middle of the circulation flow path and switches a flow path such that the liquid circulated in the circulation flow path flows out to a branch flow path to be sampled, and a controller that controls operations of the stirrer and the flow path switching unit. The controller controls the stirrer to operate at a basic speed to stir the liquid in the container during a period of circulation of the liquid in the container through the circulation flow path and controls the flow path switching unit to switch the flow path such that the liquid circulated in the circulation flow path flows out to the branch flow path to be sampled after lapse of a given time period since lowering in operation speed of the stirrer from the basic speed.

A second aspect of the present disclosure relates to a program executed by a computer that controls a sampling apparatus that samples a liquid in a container. The sampling apparatus includes a circulation mechanism that circulates the liquid in the container via a circulation flow path by leading out the liquid from the container into the circulation flow path and introducing the liquid from the circulation flow path into the container, a stirrer provided in the container, the stirrer stirring the liquid in the container, and a flow path switching unit that is provided in the middle of the circulation flow path and switches a flow path such that the liquid circulated in the circulation flow path flows out to a branch flow path to be sampled. The program, when executed by the computer, causes the computer to perform controlling the stirrer to operate at a basic speed to stir the liquid in the container during a period of circulation of the liquid in the container through the circulation flow path and controlling the flow path switching unit to switch the flow path such that the liquid circulated in the circulation flow path flows out to the branch flow path to be sampled after lapse of a given time period since lowering in operation speed of the stirrer from the basic speed.

### ADVANTAGEOUS EFFECTS OF INVENTION

According to the present disclosure, variation in amount of dissolved gas in a sampled liquid among a plurality of times of sampling is suppressed and the liquid is accurately sampled.

### BRIEF DESCRIPTION OF DRAWINGS

Fig. 1 is a block diagram illustrating a schematic configuration of an automatic pre-processing system.
Fig. 2 is a flow path diagram illustrating a flow path configuration of a sampling apparatus.
Fig. 3 is a block diagram illustrating a schematic configuration of a control device.
Fig. 4 is a perspective view of a cell culture apparatus.
Fig. 5 is a plan view of a state in which some components have been removed from the cell culture apparatus.
Fig. 6 is a partial cross-sectional view along the line VI-VI in Fig. 5.
Fig. 7 is a partial cross-sectional view along the line VII-VII in Fig. 5.
Fig. 8 is a diagram showing an internal structure of the cell culture apparatus.
Fig. 9 is a diagram showing a stirrer.
Fig. 10 is a diagram showing a state in which the stirrer has been removed from a shaft portion.
Fig. 11 is a flowchart of processing performed for sampling a culture medium in a cell culture apparatus 100 into a test tube 14 in a sampling apparatus 1.
Fig. 12 is a diagram showing an exemplary result of an amount of introduction of a liquid by introduction in accordance with the processing in Fig. 11.
Fig. 13 is a diagram showing an exemplary result of an amount of introduction of a liquid by introduction in accordance with a comparative example.

### DESCRIPTION OF EMBODIMENTS

The present embodiment will be described in detail with reference to the drawings. The same or corresponding elements in the drawings have the same reference characters allotted and description thereof will not be repeated in principle.

### <Schematic Configuration of Automatic Pre-Processing System>

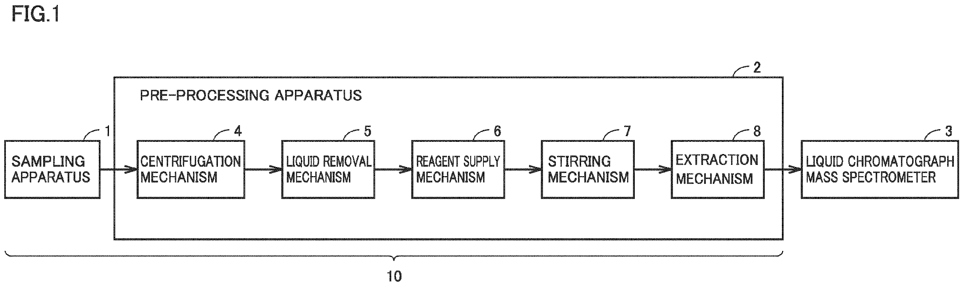

Fig. 1 is a block diagram illustrating a schematic configuration of an automatic pre-processing system 10. Automatic pre-processing system 10 is a device for automatically performing pre-processing on an analysis target. In the present embodiment, the analysis target is, for example, a cultured cell, and more specifically a bacterial cell.

Automatic pre-processing system 10 includes a sampling apparatus 1 and a pre-processing apparatus 2. Metabolites of cells pre-processed by automatic pre-processing system 10 are extracted from the cells and supplied to a liquid chromatograph mass spectrometer 3. Liquid chromatograph mass spectrometer 3 is merely an example of an analysis device for analyzing an analysis target, and it is also possible to perform analysis by using another analysis device.

Sampling apparatus 1 is an apparatus for sampling a liquid from a container (culture container). For example, cells of microorganisms and plants are cultured in a culture medium containing a medium in a container called a bioreactor. In the bioreactor, for example, a stirring member that is rotated by using magnetic force, an oxygen concentration sensor for detecting the concentration of dissolved oxygen, and the like are provided. Cells are cultured in sampling apparatus 1 by adjusting the dissolved oxygen concentration while stirring the culture medium containing the medium and the cells in the bioreactor. Detailed description of the bioreactor that functions as a cell culture apparatus will be described later.

Pre-processing apparatus 2 performs pre-processing on the cells contained in the culture medium (cultured sample) sampled from the bioreactor. In sampling apparatus 1, the culture medium containing the cells is housed in a test tube as a container (sampling container). Pre-processing apparatus 2 is provided with a centrifugation mechanism 4, a liquid removal mechanism 5, a reagent supply mechanism 6, a stirring mechanism 7, an extraction mechanism 8, and the like. Each of these mechanisms sequentially performs pre-processing on the cells contained in the culture medium in the test tube.

Centrifugation mechanism 4 applies centrifugal force to the culture medium in the test tube. The culture medium in the test tube is thus separated into a solid component that sinks to the bottom of the test tube and a liquid component that floats over the solid component, with a solid-liquid interface being defined as a boundary. The solid component is a culture material such as cultured cells. The liquid component that floats over the solid component is a supernatant separated from the culture medium.

Liquid removal mechanism 5 suctions the supernatant from the test tube. The liquid in the test tube is thus removed and the cells remain in the test tube. Reagent supply mechanism 6 supplies a reagent for extracting metabolites in cells to the cells in the test tube. A mixed solution of the cells and the reagent is thus generated in the test tube. Stirring mechanism 7 stirs the mixed solution. A suspension in which the metabolites are extracted from the cells is obtained by stirring the mixed solution.

Extraction mechanism 8 extracts some of the suspension as an extraction liquid. The extraction liquid is supplied to liquid chromatograph mass spectrometer 3.

### <Schematic Configuration of Sampling Apparatus>

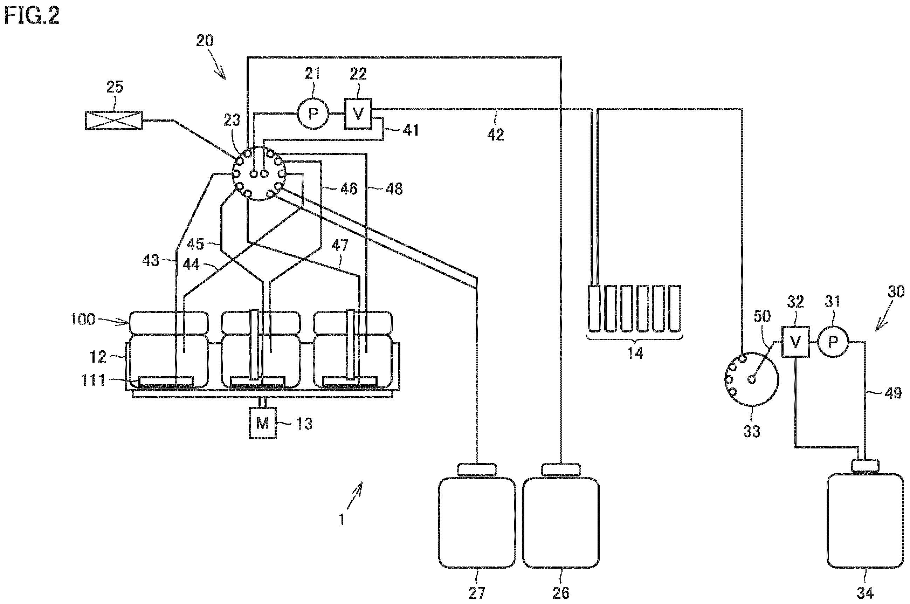

Fig. 2 is a flow path diagram illustrating a flow path configuration of sampling apparatus 1. In sampling apparatus 1, the culture medium containing the cells in cell culture apparatus 100 referred to as the bioreactor is sampled. A stirrer 111 as a stirring member that is rotated by using magnetic force is provided in cell culture apparatus 100.

Cell culture apparatus 100 is held by a holding unit 12 provided in sampling apparatus 1. In the present embodiment, one holding unit 12 can hold three cell culture apparatuses 100, and a plurality of (for example, four) such holding units 12 are provided. A configuration in which only one holding unit 12 is provided may be adopted, or a configuration in which one holding unit 12 can hold two or less or four or more cell culture apparatuses 100 may be adopted.

Cell culture apparatus 100 can carry out the culture in a state of being heated by a heater (not illustrated) provided in holding unit 12. A motor 13 for rotating a magnet (not illustrated) is connected to holding unit 12. The magnet is rotated by rotating motor 13, and stirrer 111 in each cell culture apparatus 100 can be rotated by the magnetic force thereof.

In sampling apparatus 1, the culture medium can be stirred by stirrer 111 to carry out the culture while controlling the temperature of the culture medium in cell culture apparatus 100. In sampling apparatus 1, the culture medium containing the cultured cells is sampled in a test tube 14 at arbitrary timing.

Sampling apparatus 1 is provided with a culture medium sampling mechanism 20 for sampling the culture medium in test tube 14 and a reagent sampling mechanism 30 for sampling the reagent in test tube 14. Test tube 14 houses the mixed solution in which the culture medium and the reagent are mixed with each other, is sealed by a cap (not illustrated), and is transferred to pre-processing apparatus 2.

Culture medium sampling mechanism 20 includes a pump 21 and a plurality of valves 22 and 23. Valve 23 has, for example, one pair of common ports and five pairs of selection ports (10 selection ports in total), and any one pair of selection ports is arbitrarily selected to be connected to the one pair of common ports, so that the flow path can be switched.

Pump 21 and valve 22 are provided in a flow path 41 connecting the one pair of common ports. Valve 22 constitutes a flow path switching unit (first flow path switching unit) for switching whether or not to guide the liquid in flow path 41 to a branch flow path 42 that branches with respect to flow path 41. That is, valve 22 can switch between a state in which the liquid flows between the one pair of common ports via flow path 41 and a state in which the liquid in flow path 41 is guided to branch flow path 42.

Among the five pairs of selection ports, one pair of selection ports is connected to a lead-out path 43 and an introduction path 44 that communicate with one cell culture apparatus 100, respectively. Lead-out path 43 is a flow path for leading out the culture medium in cell culture apparatus 100. On the other hand, introduction path 44 is a flow path for introducing the culture medium, which has been led out from cell culture apparatus 100 via lead-out path 43 and has been circulated through flow path 41, into cell culture apparatus 100 again. Another pair of selection ports is connected to a lead-out path 45 and an introduction path 46 that communicate with another cell culture apparatus 100, respectively. Still another pair of selection ports is connected to a lead-out path 47 and an introduction path 48 that communicate with still another cell culture apparatus 100.

In sampling apparatus 1, one of lead-out paths 43, 45, and 47 and corresponding one of introduction paths 44, 46, and 48 are caused to communicate with each other via flow path 41 and pump 21 is driven in that state, so that the culture medium in each cell culture apparatus 100 can be circulated. That is, flow path 41, each of lead-out paths 43, 45, and 47, and each of introduction paths 44, 46, and 48 constitute a circulation flow path (first circulation flow path) for circulating the culture medium in each cell culture apparatus 100.

Pump 21 constitutes a circulation mechanism (first circulation mechanism) for circulating the culture medium in each cell culture apparatus 100 via the first circulation flow path by leading out the culture medium from each cell culture apparatus 100 into the first circulation flow path and also introducing the culture medium into each cell culture apparatus 100 from the first circulation flow path.

The tip end of each of lead-out paths 43, 45, and 47 is immersed in the culture medium in corresponding cell culture apparatus 100. On the other hand, the tip end of each of introduction paths 44, 46, and 48 is located at a position separated upward from the culture medium in corresponding cell culture apparatus 100. The culture medium that is led out from cell culture apparatus 100 via each of lead-out paths 43, 45, and 47 and is circulated through flow path 41 falls from the tip end of each of introduction paths 44, 46, and 48 to be introduced into cell culture apparatus 100.

In sampling apparatus 1, in flow path 41 connecting the one pair of common ports, at least a portion where pump 21 is provided is composed of a flexible tube. Pump 21 is, for example, a tubing pump, and can feed the liquid in the flexible tube by deforming (compressing and relaxing) the tube.

When valve 22 as the first flow path switching unit provided in the middle of flow path 41 is switched, the culture medium circulated in each cell culture apparatus 100 via flow path 41 can flow out to branch flow path 42. At this time, the tip end of branch flow path 42 is arranged in test tube 14, and the culture medium is sampled in test tube 14 via branch flow path 42.

Of the two pairs of selection ports other than the three pairs of selection ports to which lead-out paths 43, 45, and 47 and introduction paths 44, 46, and 48 are connected, one pair of selection ports is connected to a cleaning liquid tank 26 and a waste liquid tank 27. The remaining one pair of selection ports is connected to a filter 25 and waste liquid tank 27, respectively. A cleaning liquid for cleaning the flow path for the culture medium is housed in cleaning liquid tank 26.

After the culture medium is sampled in test tube 14 from any of cell culture apparatuses 100, when valve 23 is switched to connect cleaning liquid tank 26 and waste liquid tank 27 to flow path 41 and pump 21 is driven in that state, the cleaning liquid in cleaning liquid tank 26 is drained into waste liquid tank 27 via flow path 41. As a result, flow path 41, valve 22, and the like can be cleaned with the cleaning liquid.

After the cleaning with the cleaning liquid, when valve 23 is switched to connect filter 25 and waste liquid tank 27 to flow path 41 and pump 21 is driven in that state, air is introduced into flow path 41 through filter 25 and discharged into waste liquid tank 27 together with water remaining in flow path 41. As a result, water can be removed from flow path 41, valve 22, and the like.

Reagent sampling mechanism 30 includes a pump 31 and a plurality of valves 32 and 33. Valve 33 has, for example, one common port and a plurality of selection ports, and any one of the selection ports is arbitrarily selected to be connected to the common port, so that the flow path can be switched.

Pump 31 and valve 32 are provided in a flow path 49 of which both ends communicate with a reagent tank 34. A reagent to be mixed with the sampled culture medium in test tube 14 is housed in reagent tank 34. Flow path 49 constitutes a circulation flow path (second circulation flow path) for circulating the reagent in reagent tank 34. Pump 31 constitutes a circulation mechanism (second circulation mechanism) for circulating the reagent in reagent tank 34 via the second circulation flow path by leading out the reagent from reagent tank 34 into the second circulation flow path and also introducing the reagent into reagent tank 34 from the second circulation flow path.

In sampling apparatus 1, in flow path 49 of which both ends are connected to reagent tank 34, at least a portion where pump 31 is provided is composed of a flexible tube. Pump 31 is, for example, a tubing pump, and can feed the reagent in the flexible tube by deforming (compressing and relaxing) the flexible tube.

Valve 32 constitutes a flow path switching unit (second flow path switching unit) for switching whether or not to guide the liquid in flow path 49 to a branch flow path 50 that branches with respect to flow path 49. That is, valve 32 can switch between a state in which the reagent in reagent tank 34 is circulated via flow path 49 and a state in which the reagent in flow path 49 is guided to branch flow path 50.

When valve 32 as the second flow path switching unit provided in the middle of flow path 49 is thus switched, the reagent circulated into reagent tank 34 via flow path 49 can flow out to branch flow path 50. Branch flow path 50 is connected to the common port of valve 33, and one of the selection ports of valve 33 is connected to the inside of test tube 14. Therefore, when the selection port connected to the inside of test tube 14 is caused to communicate with the common port, the reagent flowing out from flow path 49 to branch flow path 50 can be sampled in test tube 14.

### <Schematic Configuration of Control Device>

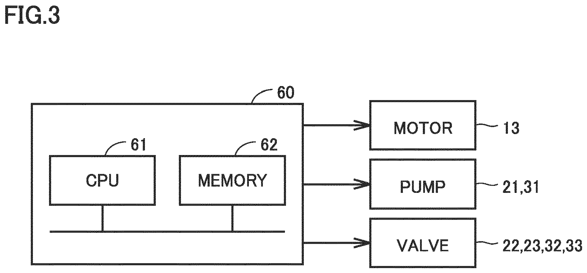

Fig. 3 is a block diagram illustrating a schematic configuration of a control device 60. Sampling apparatus 1 includes control device 60. Control device 60 includes, for example, a central processing unit (CPU) 61 and a memory 62. Memory 62 is implemented, for example, by a read only memory (ROM) and a random access memory (RAM), and various types of data other than a control program can be stored therein. CPU 61 executes a control program stored in memory 62 to control the operation of motor 13, pumps 21 and 31, valves 22, 23, 32, and 33, and the like.

Control device 60 can have the culture medium in any of cell culture apparatuses 100 circulate by driving pump 21 at a constant liquid feeding speed in a state where one of lead-out paths 43, 45, and 47 and corresponding one of introduction paths 44, 46, and 48 communicate with each other via flow path 41. Control device 60 can have valve 22 switched for a prescribed time period based on the control program to allow communication between flow path 41 and branch flow path 42 so that the culture medium in flow path 41 can be sampled in test tube 14.

Control device 60 can control an amount of sampling of the culture medium by controlling time of switching of the flow path by valve 22. That is, when the liquid feeding speed of pump 21 is known in advance, a desired amount of culture medium can accurately be sampled in test tube 14 by adjusting the time at which flow path 41 communicates with branch flow path 42.

Control device 60 can have the reagent in reagent tank 34 circulate by driving pump 31 at a constant liquid feeding speed in a state where one end of flow path 49 communicates with the other end thereof. Control device 60 can have valve 32 switched for a prescribed time period based on the control program to allow communication between flow path 49 and branch flow path 50 and have valve 33 switched to allow communication between branch flow path 50 and test tube 14, so that the culture medium in flow path 49 can be sampled in test tube 14.

Control device 60 can control an amount of sampling of the reagent by controlling time of switching of the flow path by valve 32. That is, when the liquid feeding speed of pump 31 is known in advance, a desired amount of reagent can be accurately sampled in test tube 14 by adjusting the time at which flow path 49 communicates with branch flow path 50.

### <Construction of Cell Culture Apparatus>

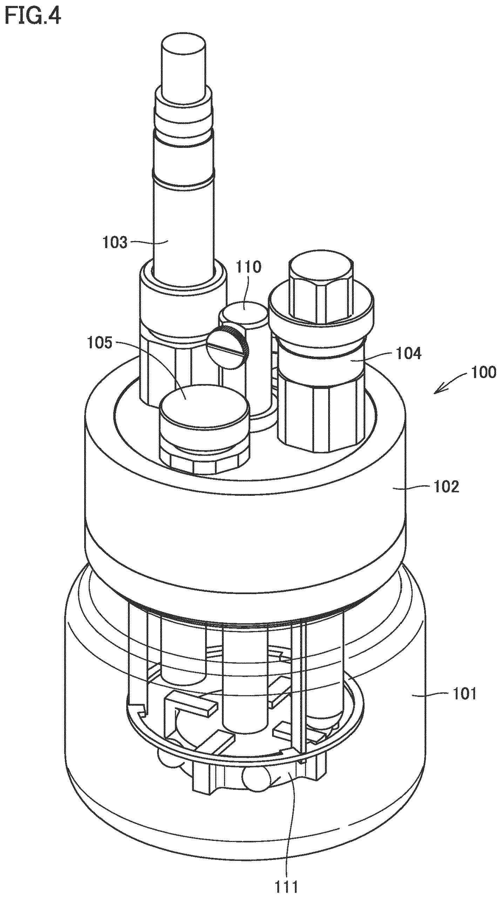

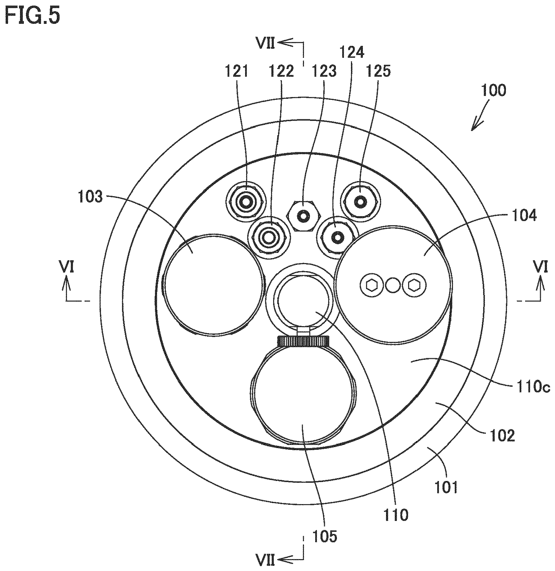

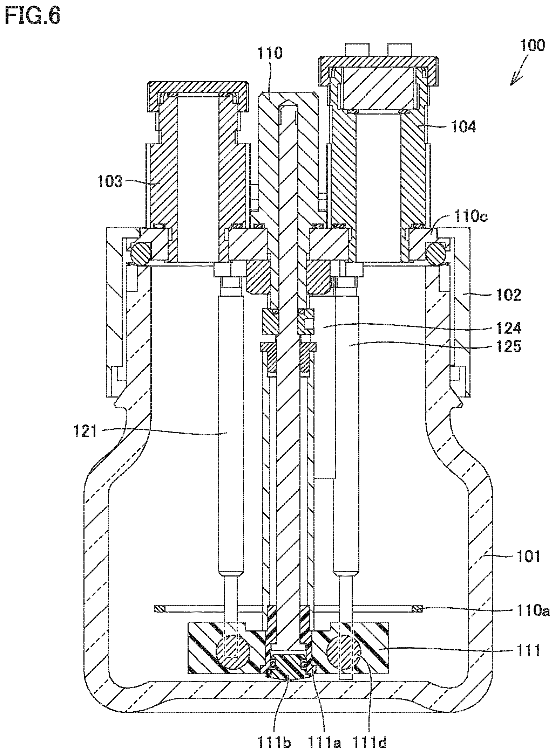

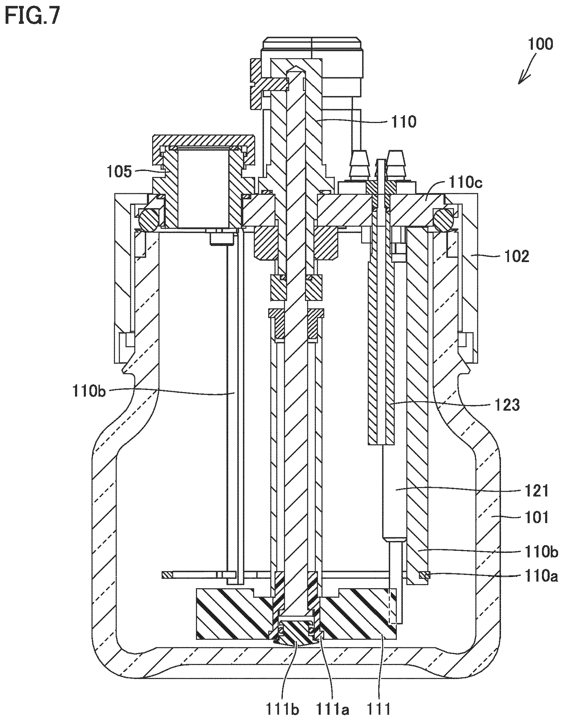

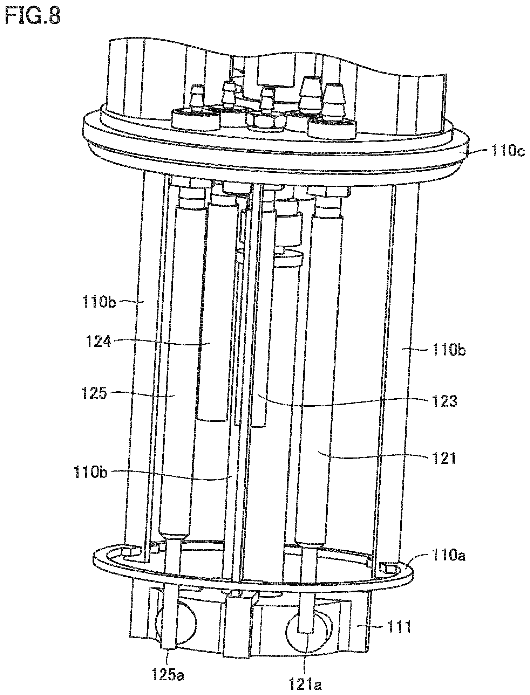

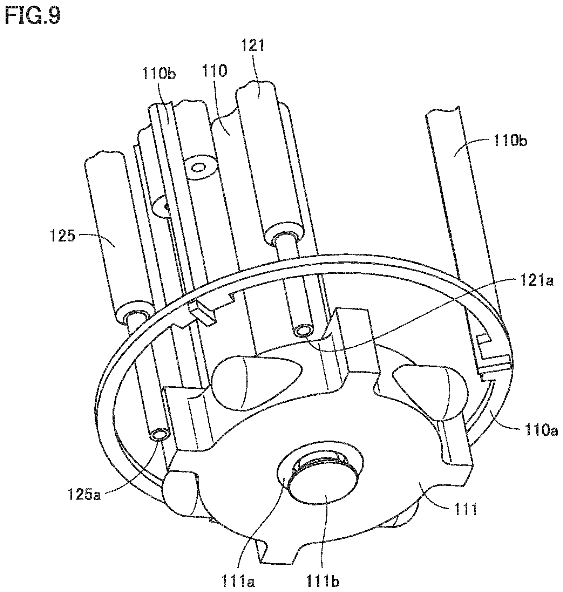

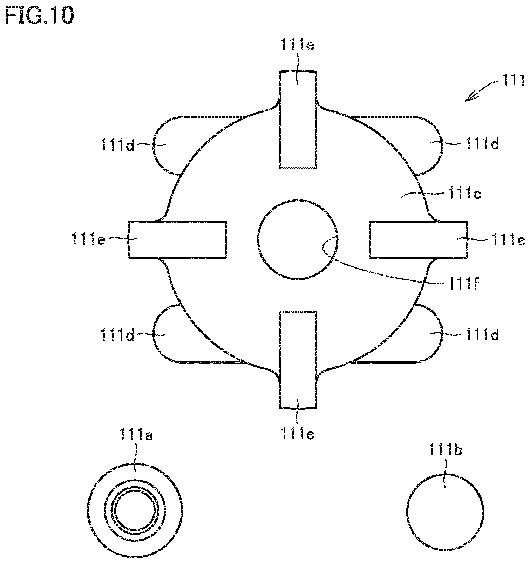

Figs. 4 to 10 illustrate a structure of cell culture apparatus 100 which is the cell culture apparatus. Fig. 4 is a perspective view of cell culture apparatus 100, Fig. 5 is a plan view of a state in which some components have been removed from cell culture apparatus 100, Fig. 6 is a partial cross-sectional view along the line VI-VI in Fig. 5, Fig. 7 is a partial cross-sectional view along the line VII-VII in Fig. 5, Fig. 8 is a diagram showing an internal structure of cell culture apparatus 100, Fig. 9 is a diagram showing stirrer 111, and Fig. 10 is a diagram showing a state in which stirrer 111 has been removed from a shaft portion 110.

As shown in Fig. 4, cell culture apparatus 100 includes a container 101, a lid portion 102, a dissolved oxygen (DO) sensor 103 connected to lid portion 102, a pH sensor 104, a cap portion 105, and shaft portion 110.

Container 101 is a transparent vessel where the culture medium containing cells of microorganisms, plants, and the like is contained. Lid portion 102 serves to hermetically seal container 101, and various components are attached thereto. DO sensor 103 is a sensor that measures a dissolved oxygen concentration in cell culture apparatus 100. pH sensor 104 is a sensor that measures a hydrogen ion concentration in the culture medium. Cap portion 105 is a lid of an opening that protrudes above cell culture apparatus 100. Shaft portion 110 is a member as a shaft of stirrer 111, around which stirrer 111 attached to a tip end thereof is rotatable.

Figs. 5 to 7 show diagrams of a state in which some components have been removed in DO sensor 103 and pH sensor 104. In the plan view in Fig. 5, five pipes connected to flexible tubes are provided between DO sensor 103 and pH sensor 104. The five pipes include an oxygen intake pipe 121, an oxygen exhaust pipe 122, a sample addition pipe 123, a suction pipe 124, and a discharge pipe 125.

Oxygen intake pipe 121 is a pipe for supply of oxygen to the culture medium, and extends to stirrer 111 located below container 101 as shown in Figs. 6 and 7. Oxygen exhaust pipe 122 is a pipe for exhaust of excessive oxygen from cell culture apparatus 100. Sample addition pipe 123 is a pipe for addition of a sample as necessary. Suction pipe 124 is a pipe connected to one of introduction paths 44, 46, and 48 described above for introduction of the culture medium into cell culture apparatus 100. Discharge pipe 125 is a pipe connected to one of lead-out paths 43, 45, and 47 described above for discharge from cell culture apparatus 100, of the culture medium in cell culture apparatus 100.

Lengths of the five pipes will be described. Oxygen exhaust pipe 122 is shortest among the five pipes and extends to a position superimposed on lid portion 102 in a vertical direction (a downward direction of upward and downward directions on the sheet plane). Sample addition pipe 123 and suction pipe 124 are substantially equal in length to each other, and they are longer than oxygen exhaust pipe 122 and extend to a central position of container 101 in the vertical direction.

Oxygen intake pipe 121 is longer than sample addition pipe 123 and suction pipe 124 and extends to a position superimposed on stirrer 111 in the vertical direction. Discharge pipe 125 is longer than oxygen intake pipe 121 and extends to a position below stirrer 111 in the vertical direction.

The five pipes are fixed to a base portion 110c on an upper surface of lid portion 102, together with shaft portion 110. Three baffle plates 110b extend downward from base portion 110c as shown in Fig. 8. Baffle plate 110b has an end fixed to an annular portion 110a. Among the five pipes, oxygen intake pipe 121 and discharge pipe 125 are fixed to annular portion 110a.

Baffle plate 110b is a member for forming turbulence that produces also an upward-downward flow with respect to a lateral flow produced by rotation of stirrer 111. Stirrer 111 has a magnet arranged in a magnet portion 111d. As shown in Figs. 6 to 9, oxygen intake pipe 121 has an end 121a located at a position superimposed on stirrer 111 in the vertical direction and discharge pipe 125 has an end 125a located below stirrer 111 in the vertical direction.

End 121a of oxygen intake pipe 121 is thus located at the position superimposed on stirrer 111 and end 125a of discharge pipe 125 is located below stirrer 111. Therefore, even when the culture medium is stirred by stirrer 111, the culture medium can be discharged to the outside of container 101 at a position where influence by oxygen bubbling caused by supply of oxygen is less likely. Therefore, cell culture apparatus 100 can accurately suck and split the sample while the amount of dissolved gas in the culture medium is maintained constant.

Stirrer 111 will now be described in detail. As shown in Fig. 10, stirrer 111 includes a main body 111c, a bearing portion 111a, and a locking portion 11 1b. Main body 111c is formed in a columnar shape provided with a hole 111f in the center, and has impeller portions 111e formed at 90° interval. Stirrer 111 includes two magnetic portions 111d that protrude from main body 111c. Magnet portion 111d is covered with a material similar to the material for main body 111c.

Main body 111c of stirrer 111 is composed of polyether ether ketone. Polyether ether ketone is generally referred to as PEEK, and will be referred to as PEEK below. A material that covers impeller portion 111e and magnet portion 111d that are formed as being integrated with main body 111c is also composed of PEEK. In contrast, bearing portion 111a and locking portion 111b are composed of polyacetal (which is abbreviated as POM).

Polyacetal and PEEK are each resin and different from each other in property. Polyacetal is used as engineering plastic excellent in strength, modulus of elasticity, and impact resistance because of presence of an amorphous portion and a crystalline portion. Since polyacetal is excellent in sliding characteristics, it is used also for a bearing component. In contrast, PEEK is categorized as super engineering plastic highest in performance. PEEK has been known as a highly reliable resin particularly excellent in heat resistance and chemical resistance among super engineering plastics.

PEEK that makes up impeller portion 111e is higher in strength and wear resistance than polyacetal that makes up bearing portion 111a. Polyacetal that makes up bearing portion 111a is self-lubricating and particularly low in coefficient of friction against metal. The inside of shaft portion 110 is formed of metal such as a stainless steel material (for example, SUS316). Polyacetal that makes up bearing portion 111a is suitable for a bearing member because it is higher in slidability with respect to metal than PEEK that makes up impeller portion 111e.

As shown in Figs. 9 and 10, stirrer 111 has bearing portion 111a inserted in hole 111f in main body 111c composed of PEEK. Bearing portion 111a is arranged as being rotatable around a shaft formed of metal in the inside of shaft portion 110 and has a bottom surface fixed by locking portion 111b.

Stirrer 111 includes bearing portion 111a composed of polyacetal which is self-lubricating and low in coefficient of friction against metal. Therefore, even when bearing portion 111a slides against shaft portion 110 for a long period of time, no chips are produced. Stirrer 111 includes impeller portion 111e composed of PEEK. Therefore, even when the stirrer rotates for a long period of time, no chips are produced between the stirrer, and oxygen intake pipe 121 and discharge pipe 125 located at a position of contact. Analysis of metabolites of microorganisms can thus be conducted in a stable manner.

In particular in the present embodiment, as shown in Figs. 6 to 8, the structure is such that baffle plate 110b has the end fixed to annular portion 110a and stirrer 111 rotates at a position below annular portion 110a. Since baffle plate 110b is thus not slid with respect to a portion that covers impeller portion 111e or magnet portion 111d of stirrer 111, production of chips can be prevented. Analysis of metabolites of microorganisms can thus be conducted in a stable manner.

### <Flow of Sampling Processing>

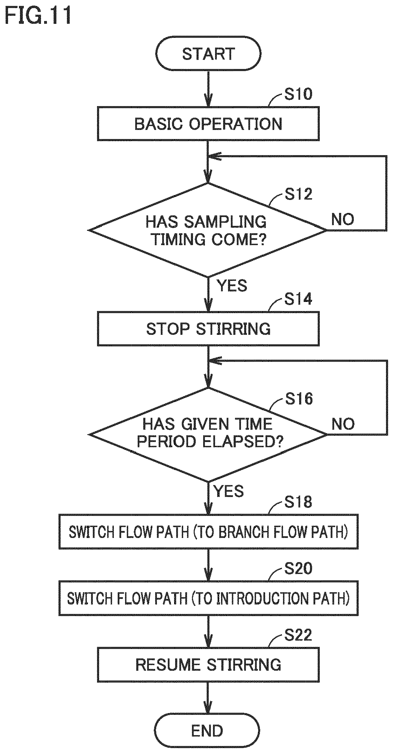

Fig. 11 is a flowchart of processing performed for sampling a culture medium in cell culture apparatus 100 into test tube 14 in sampling apparatus 1. In one implementation, in sampling apparatus 1, CPU 61 of control device 60 executes a given program to perform the processing in Fig. 11.

Control device 60 is an exemplary controller that controls operations of the stirrer (stirrer 111) and the flow path switching unit (valve 22). Control device 60 controls the operation of stirrer 111 by controlling an operation of motor 13.

The program may be stored in memory 62. In this case, memory 62 is an exemplary recording medium where the program is stored in a non-transitory manner. The program may be stored in a recording medium accessible by CPU 61 and attachable and removable to and from control device 60. In this case, the recording medium is an exemplary recording medium where the program is stored in a non-transitory manner.

The processing in Fig. 11 is performed for each test tube 14. For the sake of convenience of description, sampling from leftmost cell culture apparatus 100 among three cell culture apparatuses 100 shown in Fig. 2 will be described below. Specifically, in this description, flow path 41, together with lead-out path 43 and introduction path 44, defines the first circulation flow path. A flow of the processing will be described below with reference to Fig. 11.

In step S10, control device 60 controls cell culture apparatus 100 to perform a basic operation. The basic operation includes circulation of the culture medium through the first circulation flow path and circulation of the reagent through the second circulation flow path. Circulation of the culture medium through the first circulation flow path includes rotation of motor 13 to rotate stirrer 111 to thereby stir the culture medium in cell culture apparatus 100.

In step S12, control device 60 determines whether or not timing of sampling of the culture medium into test tube 14 has come. Control device 60 repeats determination in step S12 until it determines that the timing has come (NO in step S12). When control device 60 determines that the timing has come (YES in step S12), it has control proceed to step S14.

In step S14, control device 60 controls motor 13 to stop rotation to thereby stop rotation of stirrer 111. Stirring of the culture medium in cell culture apparatus 100 is thus stopped.

In step S16, control device 60 determines whether or not a given time period has elapsed since stirring was stopped in step S14. Control device 60 continues control in step S16 until it determines that the given time period has elapsed (NO in step S16), and when it determines that the given time period has elapsed (YES in step S16), it has control proceed to step S18.

In step S18, control device 60 controls valve 22 to switch the flow path to guide the liquid in flow path 41 to branch flow path 42.

In step S20, control device 60 controls valve 22 to switch the flow path to guide the liquid in flow path 41 to introduction path 44 after a specific time period has elapsed since it controlled valve 22 to switch the flow path in step S18. The specific time period in step S20 means a time period corresponding to a given amount of sampling.

In step S22, control device 60 controls motor 13 to resume rotation to thereby resume stirring of the culture medium in cell culture apparatus 100. Thereafter, control device 60 quits the processing in Fig. 11.

In the processing described above with reference to Fig. 11, in step S18, valve 22 switches the flow path for sampling into test tube 14. Stirring in cell culture apparatus 100 is stopped the given time period before switching of the flow path in step S18 (step S14). Specifically, after lapse of the given time period since stop of stirring in step S14, the flow path is switched and sampling is started in step S18.

As a result of sampling after stirring was stopped for the "given time period" in cell culture apparatus 100, such a situation as variation in amount of dissolved gas in the sampled culture medium among a plurality of times of sampling is suppressed. When the "given time period" is too short, variation in amount of dissolved gas among the plurality of times of sampling is not sufficiently suppressed. When the "given time period" is too long, on the other hand, such a situation as occurrence of uneven culture in the sampled culture medium among the plurality of times of sampling is assumed. In this sense, in one implementation, the "given time period" from two minutes to ten minutes may be set. In another implementation, the "given time period" from three minutes to seven minutes may be set.

So long as occurrence of variation in amount of dissolved gas among the plurality of times of sampling is suppressed, stirring does not have to completely be stopped. Specifically, control device 60 may set a rotation speed of stirrer 111 lower than the rotation speed in the basic operation in step S10, instead of stopping rotation of stirrer 111 in step S14. Control device 60 lowers the rotation speed of stirrer 111 by lowering the rotation speed of motor 13. The rotation speed of stirrer 111 in step S10 is also referred to as a "basic speed," and it is a speed set for circulation of the culture medium.

In one implementation, in step S14, control device 60 lowers the rotation speed of stirrer 111 to approximately 1/10 of the basic speed. In this case, after sampling is completed and the flow path is switched in step S20, control device 60 sets the rotation speed of stirrer 111 back to the rotation speed in step S10.

### <Equalization of Amount of Introduction into Test Tube>

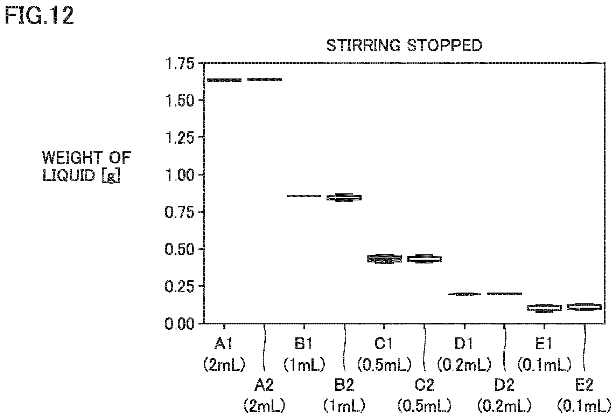

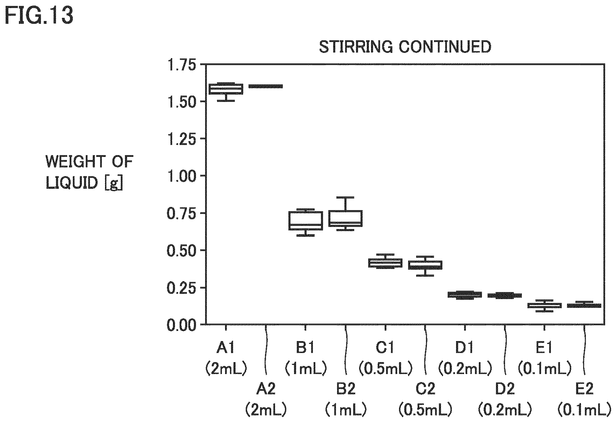

Fig. 12 is a diagram showing an exemplary result of an amount of introduction of a liquid by introduction of the liquid in accordance with the processing in Fig. 11. Fig. 13 is a diagram showing an exemplary result of an amount of introduction of a liquid by introduction of the liquid in accordance with a comparative example. Results in each of Figs. 12 and 13 are results when pure water is adopted as the liquid to be introduced from cell culture apparatus 100 into test tube 14. In a graph in each of Figs. 12 and 13, the ordinate represents a weight of the liquid introduced in test tube 14.

The result shown in Fig. 12 is the result when stirring by stirrer 111 is stopped a given time period before introduction of the liquid from cell culture apparatus 100 into test tube 14 as described with reference to Fig. 11. The result shown in Fig. 13, on the other hand, is the result when stirring by stirrer 111 is continued around the time of introduction of the liquid from cell culture apparatus 100 into test tube 14.

Each of Figs. 12 and 13 shows a range of an amount of introduction (a maximum value and a minimum value) in introduction of the liquid six times, for each of ten groups (A1 to E2). The groups are different from one another in target volume of introduction of the liquid. The target volume of introduction for groups A1 an A2 is set to 2 mL, the target volume of introduction for groups B1 and B2 is set to 1 mL, the target volume of introduction for groups C1 and C2 is set to 0.5 mL, the target volume of introduction for groups D1 and D2 is set to 0.2 mL, and the target volume of introduction for groups E1 and E2 is set to 0.1 mL.

The result in Fig. 13 is larger than the result in Fig. 12 in difference between the maximum value and the minimum value of the weight of the liquid introduced in test tube 14 in each group.

For example, in Fig. 13, in group A1, the maximum value is 1.65 g, the minimum value is 1.50 g, and the difference between the maximum value and the minimum value is 0.15 g. Since a median value is 1.575 g, 0.15 g which is the difference between the maximum value and the minimum value is approximately 10% of the median value 1.575 g, which is a relatively large value.

In Fig. 13, in group B2, the maximum value is 0.90 g, the minimum value is 0.60 g, and the difference between the maximum value and the minimum value is 0.30 g. Since the median value is 0.75 g, 0.30 g which is the difference between the maximum value and the minimum value is approximately 43% of the median value 0.75 g, which is a relatively large value.

In the result in Fig. 12, on the other hand, the difference between the maximum value and the minimum value of the weight of the liquid introduced into test tube 14 is small in each group. In other words, in the result in Fig. 12, variation in weight of the liquid introduced into test tube 14 is substantially not observed in each group. Specifically, when stirring by stirrer 111 is stopped a given time period before introduction of the liquid from cell culture apparatus 100 into test tube 14 as described with reference to Fig. 11, variation in ratio of gas in a suctioned solution is suppressed and accuracy of the suctioned solution may thus be controlled. Such an effect may be expected not only when stirring by stirrer 111 is completely stopped but also when the rotation speed for stirring by stirrer 111 is lowered.

### [Aspects]

Illustrative embodiments described above are understood by a person skilled in the art as specific examples of aspects below.

(Clause 1) A sampling apparatus according to one aspect may be a sampling apparatus that samples a liquid in a container, the sampling apparatus including a circulation mechanism that circulates the liquid in the container via a circulation flow path by leading out the liquid from the container into the circulation flow path and introducing the liquid from the circulation flow path into the container, a stirrer provided in the container, the stirrer stirring the liquid in the container, a flow path switching unit that is provided in the middle of the circulation flow path and switches a flow path such that the liquid circulated in the circulation flow path flows out to a branch flow path to be sampled, and a controller that controls operations of the stirrer and the flow path switching unit. The controller may control the stirrer to operate at a basic speed to stir the liquid in the container during a period of circulation of the liquid in the container through the circulation flow path and may control the flow path switching unit to switch the flow path such that the liquid circulated in the circulation flow path flows out to the branch flow path to be sampled after lapse of a given time period since lowering in operation speed of the stirrer from the basic speed.

According to the sampling apparatus described in Clause 1, before the flow path is switched for sampling, the operation speed of the stirrer that stirs the liquid in the container is lowered for the given time period. Variation in amount of dissolved gas in the sampled liquid among a plurality of times of sampling is thus suppressed and the liquid is accurately sampled.

(Clause 2) In the sampling apparatus described in Clause 1, lowering the operation speed of the stirrer from the basic speed may include stopping the stirrer.

According to the sampling apparatus described in Clause 2, variation in amount of dissolved gas in the sampled liquid among the plurality of times of sampling can more reliably be suppressed.

(Clause 3) In the sampling apparatus described in Clause 1 or 2, a length of the given time period may be from two minutes to ten minutes.

According to the sampling apparatus described in Clause 3, such a situation as insufficiently suppressed variation in amount of dissolved gas among a plurality of times of sampling due to too short a "given time period" is avoided. Furthermore, such a situation as occurrence of uneven culture in the sampled culture medium for each time of sampling due to too long a "given time period" is avoided.

(Clause 4) In the sampling apparatus described in Clause 1 or 2, a length of the given time period may be from three minutes to seven minutes.

According to the sampling apparatus described in Clause 4, such a situation as insufficiently suppressed variation in amount of dissolved gas among a plurality of times of sampling due to too short a "given time period" is more reliably avoided. Furthermore, such a situation as occurrence of uneven culture in the sampled culture medium for each time of sampling due to too long a "given time period" is more reliably avoided.

(Clause 5) A program according to one aspect may be a program executed by a computer that controls a sampling apparatus that samples a liquid in a container. The sampling apparatus may include a circulation mechanism that circulates the liquid in the container via a circulation flow path by leading out the liquid from the container into the circulation flow path and introducing the liquid from the circulation flow path into the container, a stirrer provided in the container, the stirrer stirring the liquid in the container, and a flow path switching unit that is provided in the middle of the circulation flow path and switches a flow path such that the liquid circulated in the circulation flow path flows out to a branch flow path to be sampled. The program, when executed by the computer, may cause the computer to perform controlling the stirrer to operate at a basic speed to stir the liquid in the container during a period of circulation of the liquid in the container through the circulation flow path and controlling the flow path switching unit to switch the flow path such that the liquid circulated in the circulation flow path flows out to the branch flow path to be sampled after lapse of a given time period since lowering in operation speed of the stirrer from the basic speed.

According to the program described in Clause 5, before the flow path is switched for sampling, the operation speed of the stirrer that stirs the liquid in the container is lowered for the given time period. Variation in amount of dissolved gas in the sampled liquid among a plurality of times of sampling is thus suppressed and the liquid is accurately sampled.

(Clause 6) In the program described in Clause 5, lowering the operation speed of the stirrer from the basic speed may include stopping the stirrer.

According to the program described in Clause 6, variation in amount of dissolved gas in the sampled liquid among a plurality of times of sampling can more reliably be suppressed.

(Clause 7) In the program described in Clause 5 or 6, a length of the given time period may be from two minutes to ten minutes.

According to the program described in Clause 7, such a situation as insufficiently suppressed variation in amount of dissolved gas among a plurality of times of sampling due to too short a "given time period" is avoided. Furthermore, such a situation as occurrence of uneven culture in the sampled culture medium for each time of sampling due to too long a "given time period" is avoided.

(Clause 8) In the program described in Clause 5 or 6, a length of the given time period may be from three minutes to seven minutes.

According to the program described in Clause 8, such a situation as insufficiently suppressed variation in amount of dissolved gas among a plurality of times of sampling due to too short a "given time period" is more reliably avoided. Furthermore, such a situation as occurrence of uneven culture in the sampled culture medium for each time of sampling due to too long a "given time period" is more reliably avoided.

It should be understood that the embodiment disclosed herein is illustrative and non-restrictive in every respect. The scope of the present disclosure is defined by the terms of the claims rather than the description of the embodiment above and is intended to include any modifications within the scope and meaning equivalent to the terms of the claims.

### REFERENCE SIGNS LIST

1 sampling apparatus; 2 pre-processing apparatus; 3 liquid chromatograph mass spectrometer; 4 centrifugation mechanism; 5 liquid removal mechanism; 6 reagent supply mechanism; 7 stirring mechanism; 8 extraction mechanism; 10 pre-processing system; 12 holding unit; 13 motor; 14 test tube; 20 culture medium sampling mechanism; 21, 31 pump; 22, 23, 32, 33 valve; 25 filter; 26 cleaning liquid tank; 27 waste liquid tank; 30 reagent sampling mechanism; 34 reagent tank; 41, 42, 49, 50 flow path; 43, 45, 47 lead-out path; 44, 46, 48 introduction path; 60 control device; 61 CPU; 62 memory; 100 cell culture apparatus; 101 container; 102 lid portion; 103 DO sensor; 104 pH sensor; 105 cap portion; 110 shaft portion; 110a annular portion; 110b baffle plate; 110c base portion; 111 stirrer; 111a bearing portion; 111b locking portion; 111c main body; 111d magnet portion; 111e impeller portion; 121 oxygen intake pipe; 121a, 125a end; 122 oxygen exhaust pipe; 123 sample addition pipe; 124 suction pipe; 125 discharge pipe

## Claims

1. A sampling apparatus that samples a liquid in a container, the sampling apparatus comprising:
a circulation mechanism that circulates the liquid in the container via a circulation flow path by leading out the liquid from the container into the circulation flow path and introducing the liquid from the circulation flow path into the container;
a stirrer provided in the container, the stirrer stirring the liquid in the container;
a flow path switching unit that is provided in middle of the circulation flow path and switches a flow path such that the liquid circulated in the circulation flow path flows out to a branch flow path to be sampled; and
a controller that controls operations of the stirrer and the flow path switching unit, wherein
the controller
controls the stirrer to operate at a basic speed to stir the liquid in the container during a period of circulation of the liquid in the container through the circulation flow path, and
controls the flow path switching unit to switch the flow path such that the liquid circulated in the circulation flow path flows out to the branch flow path to be sampled after lapse of a given time period since lowering in operation speed of the stirrer from the basic speed.

2. The sampling apparatus according to claim 1, wherein
lowering the operation speed of the stirrer from the basic speed includes stopping the stirrer.

3. The sampling apparatus according to claim 1 or 2, wherein
a length of the given time period is from two minutes to ten minutes.

4. The sampling apparatus according to claim 1 or 2, wherein
a length of the given time period is from three minutes to seven minutes.

5. A program executed by a computer that controls a sampling apparatus that samples a liquid in a container, the sampling apparatus including a circulation mechanism that circulates the liquid in the container via a circulation flow path by leading out the liquid from the container into the circulation flow path and introducing the liquid from the circulation flow path into the container, a stirrer provided in the container, the stirrer stirring the liquid in the container, and a flow path switching unit that is provided in middle of the circulation flow path and switches a flow path such that the liquid circulated in the circulation flow path flows out to a branch flow path to be sampled, the program, when executed by the computer, causing the computer to perform:
controlling the stirrer to operate at a basic speed to stir the liquid in the container during a period of circulation of the liquid in the container through the circulation flow path; and
controlling the flow path switching unit to switch the flow path such that the liquid circulated in the circulation flow path flows out to the branch flow path to be sampled after lapse of a given time period since lowering in operation speed of the stirrer from the basic speed.

6. The program according to claim 5, wherein
lowering the operation speed of the stirrer from the basic speed includes stopping the stirrer.

7. The program according to claim 5 or 6, wherein
a length of the given time period is from two minutes to ten minutes.

8. The program according to claim 5 or 6, wherein
a length of the given time period is from three minutes to seven minutes.
